(19) **Europäisches Patentamt**
**European Patent Office**
**Office européen des brevets**

(11) **EP 4 114 518 B1**

(12) **FASCICULE DE BREVET EUROPEEN**

(45) Date de publication et mention
de la délivrance du brevet:
**01.10.2025 Bulletin 2025/40**

(21) Numéro de dépôt: **21708017.5**

(22) Date de dépôt: **02.03.2021**

(51) Classification Internationale des Brevets (IPC):
***A61N 7/00*** *(2006.01)* ***G01N 29/032*** *(2006.01)*
***G01M 3/40*** *(2006.01)*

(52) Classification Coopérative des Brevets (CPC):
**A61N 7/00; G01M 3/40; G01N 29/032;**
A61N 2007/0073; A61N 2007/0078;
A61N 2007/0082; G01N 2291/015;
G01N 2291/02433; G01N 2291/02475;
G01N 2291/02483

(86) Numéro de dépôt international:
**PCT/EP2021/055140**

(87) Numéro de publication internationale:
**WO 2021/175828 (10.09.2021 Gazette 2021/36)**

(54) **SYSTÈME POUR LA DÉTECTION D'UN DÉFAUT DE COUPLAGE ACOUSTIQUE ENTRE UN DISPOSITIF ULTRASONORE ET UN TISSU À TRAITER**

SYSTEM ZUR ERKENNUNG EINES FEHLERS IN DER AKUSTISCHEN KOPPLUNG ZWISCHEN EINER ULTRASCHALLVORRICHTUNG UND EINEM ZU BEHANDELNDEN GEWEBE

SYSTEM FOR DETECTING A FAULT IN ACOUSTIC COUPLING BETWEEN AN ULTRASONIC DEVICE AND A TISSUE TO BE TREATED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.03.2020 FR 2002062**

(43) Date de publication de la demande:
**11.01.2023 Bulletin 2023/02**

(73) Titulaire: **Carthera**
**69008 Lyon (FR)**

(72) Inventeurs:
• **BOUCHOUX, Guillaume**
**69100 VILLEURBANNE (FR)**

• **CHOLVY, Matthieu**
**38550 PEAGE DE ROUSSILLON (FR)**
• **MARTIN, Cyril**
**69003 LYON (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A1- 3 216 410       EP-A2- 0 050 040**
**WO-A1-2018/185767    WO-A1-2020/013868**
**FR-A1- 3 053 597       JP-A- H09 297 160**

# EP 4 114 518 B1

**Description**

## DOMAINE DE L'INVENTION

La présente invention concerne le domaine technique général des dispositifs ultrasonores - par exemple des dispositifs intracorporels ou implantables - destinés à être raccordés électriquement à une unité de commande distante.

De tels dispositifs peuvent notamment être implantés chez l'homme et les mammifères pour aider un praticien dans l'établissement d'un diagnostic et/ou pour traiter une pathologie.

## ARRIERE PLAN DE L'INVENTION

On connaît du document WO 2018/007500 un appareil de traitement d'affections du cerveau. En référence à la figure 1, un tel appareil se compose :

- d'un dispositif ultrasonore 1 réalisé en matériau non ferromagnétique,
- d'une unité de commande 2 distante du dispositif ultrasonore 1, et
- de moyens de connexion entre le dispositif ultrasonore 1 et l'unité de commande 2.

Le dispositif ultrasonore 1 est destiné à être positionné dans un trou de trépan effectué dans un crâne d'un patient. Il est avantageusement compatible avec la technique d'Imagerie par Résonance Magnétique (IRM), et comprend :

- au moins un transducteur 12 pour la génération d'ondes ultrasonores de traitement d'une affection cérébrale,
- des moyens de fixation 13 pour fixer le transducteur 12 dans le crâne du patient,
- une (ou plusieurs) borne(s) 14 de connexion électrique destinée(s) à coopérer avec les moyens de connexion.

L'unité de commande 2 est destinée à alimenter en énergie électrique le dispositif ultrasonore 1, et à régler ses paramètres de fonctionnement.

Les moyens de connexion sont destinés à relier électriquement le dispositif ultrasonore 1 à l'unité de commande 2. Ils comprennent généralement :

- un (ou plusieurs) câble(s) 31 de connexion électrique dont l'une des extrémités est reliée à l'unité de commande, et
- une (ou plusieurs) aiguille(s) transdermique(s) 32 raccordée(s) à l'autre extrémité du câble 31.

Le principe de fonctionnement de cet appareil est le suivant. Une fois le dispositif ultrasonore 1 implanté dans le crâne du patient, une succession de séances de traitement lui sont prodiguées pour traiter la pathologie qui l'affecte. A chaque nouvelle séance de traitement, le dispositif ultrasonore 1 est relié à l'unité de commande 2 par l'intermédiaire des moyens de connexion.

Le praticien relie le câble 31 à l'unité de commande 2 puis il insère l'aiguille 32 à travers la peau du patient jusqu'à la borne 14 du dispositif ultrasonore.

Une fois l'extrémité de l'aiguille 32 connectée à la borne 14, l'unité de commande 2 peut être activée pour alimenter le dispositif ultrasonore 1 en énergie électrique.

Le procédé de détection décrit dans WO 2018/007500 propose, préalablement à la mise en œuvre du traitement, de vérifier la qualité du raccordement électrique entre le dispositif ultrasonore 1 et l'unité de commande 2.

Plus précisément, le système et le procédé décrits dans WO 2018/007500 permettent de détecter différents types de raccordement électriques défectueux tel que :

- l'absence de connexion électrique entre le câble 31 et l'unité de commande 2,
- l'absence de connexion électrique entre l'aiguille transdermique et la borne 14.

Le fait de vérifier la qualité du raccordement électrique entre le dispositif ultrasonore 1 et l'unité de commande 2 préalablement à la mise en œuvre du traitement permet de limiter les risques d'inefficacité du traitement.

Toutefois, d'autres paramètres peuvent influer sur l'efficacité du traitement, et notamment la qualité du couplage acoustique entre le dispositif ultrasonore 1 et le tissu à traiter.

Le document WO 2020/013868 concerne un appareil de caractérisation de propriété d'une membrane ductile, d'une surface et d'une sous-surface. Un processeur de signal ultrasonore utilise un générateur d'excitation pour provoquer le déplacement d'une membrane ou d'une surface pendant qu'une série d'impulsions ultrasonores sont appliquées à la membrane ou la surface. Des différences de phase entre un signal émis et un signal reçu sont examinées afin de déterminer le mouvement de la membrane ou de la surface en réponse à l'excitation appliquée. Un examen de la réponse de phase de la membrane ou de la surface permet de déterminer si le type de fluide derrière la membrane ou la surface est l'un des fluides suivants : aucun fluide, un fluide sérique ou un fluide purulent.

Le document WO 2018/185767 décrit un système permettant d'améliorer la fonction rénale par application d'énergie acoustique et/ou ultrasonore à un ou plusieurs des reins. Le procédé comprend une distribution d'énergie focalisée ou non focalisée, éventuellement dans une approche non invasive, qui pourrait également être mise en œuvre de manière invasive et/ou par un dispositif implantable.

Le document EP 0 050 040 concerne un appareil pour détecter des impuretés dans un liquide tel que le sang. Une quantité minimale de rayonnement Doppler ultrasonore à bande passante étroite pour maximiser la sensibilité est dirigée à travers un transducteur focalisé dans le champ lointain et positionné à un angle par rapport à un flux de liquide, tel que du sang, à surveiller. Le rayonnement réfléchi reçu au niveau du transducteur, dû aux agents de rétrodiffusion dans le liquide, est utilisé pour générer un signal de puissance de rétrodiffusion dont le niveau dépend des agents de diffusion dans le liquide. Ce signal est utilisé pour déterminer la présence d'impuretés dans le liquide, qui, dans le cas des particules de carbone dans le sang, fournissent un niveau de puissance moyen plus élevé que les agents de diffusion dans le sang pur.

Un but de la présente invention est de proposer un procédé et un système permettant au praticien de détecter un éventuel défaut dans la qualité du couplage acoustique entre :

- un dispositif ultrasonore, et
- un tissu à traiter.

Le dispositif ultrasonore peut également subir des dégradations au cours du temps. Notamment, le fonctionnement d'un (ou plusieurs) transducteur du dispositif peut être défaillant, par exemple si une connexion électrique d'un (ou plusieurs) transducteur(s) est altérée (court-circuit ou circuit ouvert), par exemple suite à la désolidarisation d'une (ou de plusieurs) patte(s) de connexion d'un (ou de plusieurs) transducteur(s).

Un autre but de l'invention est de proposer un procédé et un système permettant au praticien de détecter un défaut de fonctionnement d'un (ou de plusieurs) transducteur(s) du dispositif ultrasonore.

## BREVE DESCRIPTION DE L'INVENTION

L'invention est définie par l'objet de la revendication indépendante 1. D'autres modes de réalisation sont divulgués dans les revendications dépendantes. Les méthodes de traitement sont divulguées uniquement à titre d'illustration de la manière dont le dispositif pourrait être utilisé et ne sont pas revendiquées.

A cet effet, l'invention propose un appareil de traitement d'une pathologie comprenant

- un dispositif ultrasonore comportant au moins un transducteur apte à générer des ondes ultrasonores, le transducteur ayant une face avant destinée à être positionnée en regard d'un milieu cible,
- une unité de commande distante pour déterminer et contrôler des paramètres de fonctionnement du dispositif ultrasonore, et lui délivrer de l'électricité pendant au moins un cycle de traitement, chaque cycle de traitement étant précédé d'un cycle d'attente,
- des moyens de connexion électrique entre le dispositif ultrasonore et l'unité de commande,

***remarquable en ce que*** l'unité de commande est programmée pour mettre en œuvre une phase d'estimation de la qualité d'un couplage acoustique entre le dispositif ultrasonore et le milieu cible, ladite phase d'estimation comprenant :

- l'émission par l'unité de commande, d'au moins un signal de contrôle, chaque signal de contrôle ayant un fréquence respective,
- la mesure par l'unité de commande, d'au moins un signal réfléchi, chaque signal réfléchi correspondant à un signal de contrôle respectif,
- le traitement du signal réfléchi pour détecter :

    ○ soit la présence d'un liquide dans le dispositif ultrasonore,
    ○ soit la présence d'un matériau réfléchissant, tel qu'une bulle de gaz, entre ledit et au moins un transducteur et le milieu cible.

Les autres aspects de la présente invention sont les suivants :

- la phase d'estimation peut comprendre une étape de détection de la présence de liquide dans le dispositif ultrasonore, ladite étape incluant les sous-étapes suivantes :

∘ l'émission par l'unité de commande, d'un signal de contrôle de courant de fuite à une fréquence de contrôle de courant de fuite,
∘ la mesure par l'unité de commande, d'un signal réfléchi de contrôle de courant de fuite correspondant à la portion du signal de contrôle de courant de fuite n'ayant pas été absorbée par le dispositif ultrasonore,
∘ le traitement du signal réfléchi de contrôle de courant de fuite pour détecter la présence d'un liquide dans le dispositif ultrasonore ; Des aspects préférés mais non limitatifs de la présente invention sont les suivants:

- la fréquence de contrôle de courant de fuite peut être une fréquence n'appartenant pas à une plage de fréquence de fonctionnement du transducteur, notamment une fréquence de l'ordre de 600 kHz pour un transducteur dont la fréquence de travail est égale à 1 MHz ;
- la phase d'estimation peut comprendre une étape de détection de la présence d'une bulle de gaz, ladite étape incluant les sous-étapes suivantes :

  ∘ l'émission par l'unité de commande, d'un signal de contrôle de gaz à une fréquence de contrôle de gaz,
  ∘ la mesure par l'unité de commande, d'un signal réfléchi de contrôle de gaz correspondant à la portion du signal de contrôle de gaz n'ayant pas été absorbée par le dispositif ultrasonore,
  ∘ le traitement du signal réfléchi de contrôle de gaz pour détecter la présence d'une bulle de gaz entre le transducteur et le milieu cible.

- la fréquence de contrôle d'une bulle de gaz peut être une fréquence appartenant à une plage de fréquence de fonctionnement du transducteur, plus précisément une fréquence supérieure à 90% d'une fréquence de travail du transducteur, notamment une fréquence de l'ordre de 962kHz pour un transducteur dont la fréquence de travail est égale à 1 MHz ;
- l'étape de détection de la présence d'une bulle de gaz peut être mise en œuvre pour chaque transducteur pendant au moins un cycle d'attente, ladite étape incluant en outre une étape consistant à :

  ∘ activer chaque transducteur pour lequel aucune bulle de gaz n'a été détectée, les transducteurs activés étant aptes à être alimentés en énergie électrique pour la génération d'ondes ultrasonores de traitement pendant au moins un cycle de traitement postérieur audit et au moins un cycle d'attente,
  ∘ désactiver chaque transducteur pour lequel une bulle de gaz a été détectée, les transducteurs désactivés n'étant pas alimentés en énergie électrique lors du cycle de traitement postérieur audit et au moins un cycle d'attente.

- chaque séance de traitement peut comprendre une pluralité de cycles de traitement pendant lesquels le dispositif émet des ondes ultrasonores de traitement vers un tissu à traiter, chaque cycle de traitement étant précédé d'un cycle d'attente, l'unité de commande étant programmée pour mettre en œuvre :

  ∘ l'étape de détection de la présence d'une bulle de gaz pendant chaque cycle d'attente,
  ∘ l'étape de détection de la présence d'un liquide pendant chaque cycle de traitement ;

- les étapes de détection de la présence de liquide et de gaz peuvent être mises en œuvre séquentiellement, l'étape de détection de la présence de liquide étant mise en œuvre postérieurement à l'étape de détection de la présence d'une bulle de gaz ;
- le dispositif ultrasonore peut comporter un boitier dans lequel chaque transducteur est logé, le boitier incluant un fond en regard de la face avant de chaque transducteur, le fond étant constitué en Poly-Ether-Ether-Ketone, l'épaisseur du fond étant, pour une fréquence de travail du transducteur égale à 1MHz, comprise entre 0.3 mm et 0.8 mm, préférentiellement comprise entre 0.3mm et 0.6 mm, et encore plus préférentiellement sensiblement égale à 0,4mm±0.05 mm.

La phase d'estimation peut comprendre une étape de détection du fonctionnement de chaque transducteur. En particulier de détection d'un court-circuit ou d'un circuit ouvert. Pour cela l'unité de commande envoie vers chaque transducteur une tension à fréquence $F_0$, cette fréquence étant avantageusement nulle (tension continue)
L'invention propose également un dispositif ultrasonore implantable comportant au moins un transducteur apte à générer des ondes ultrasonores, le transducteur comportant :

- au moins un élément électro-acoustiques constitué dans un matériau piézo-électrique, et
- un boitier incluant un fond, au moins une paroi latérale et une paroi supérieure, le boitier formant un logement étanche destiné à contenir ledit et au moins un élément électroacoustique,

**remarquable en ce que** le matériau constituant le fond du boitier est du Poly-Ether-Ether-Ketone (PEEK).

Des aspects préférés mais non limitatifs de la présente invention sont les suivants :

- l'élément électro-acoustique comporte une face avant destinée à être positionnée en regard du tissu à traiter et une face arrière opposée à la face avant, le transducteur comprenant une couche réfléchissant les ondes acoustiques, telle qu'une couche d'air, ladite couche s'étendant sur la face arrière de l'élément électro-acoustique ;
  il sera entendu dans la suite que lorsqu'une couche A est mentionnée comme étant *« s'étendant sur »* une couche B, celle-ci peut être directement sur la couche B, ou peut être située au-dessus de la couche B et séparée de ladite couche B par une ou plusieurs couches intermédiaires jouant un rôle acoustique négligeable à la fréquence de travail du transducteur ;
- la face avant de l'élément électro-acoustique est en contact avec le fond du boitier ;
  il sera entendu dans la suite que lorsqu'une couche A est mentionnée comme étant *« en contact avec »* une couche B, celle-ci peut être directement en contact avec la couche B, ou peut être séparée de ladite couche B par une ou plusieurs couches intermédiaires jouant un rôle acoustique négligeable à la fréquence de travail du transducteur ;
- le matériau constituant ladite et au moins une paroi latérale et le couvercle du boitier est également du Poly-Ether-Ether-Ketone (PEEK) ;
- l'épaisseur du fond du boitier satisfait la relation suivante :

$$E_{Fond} = (V_{son} / 4F_{Travail}) \times (0.8 \pm 0.4) \,,$$

Où :

- $E_{Fond}$ correspond à l'épaisseur du fond du boitier (en mm),
- $V_{son}$ correspond à la vitesse du son dans le matériau constituant le fond du boitier, et
- $F_{Travail}$ correspond à la fréquence de travail du transducteur 12 (en MHz), ladite fréquence de travail étant choisie dans la bande de fréquence utile du transducteur 12.

- pour une fréquence de travail de l'ordre de 1MHz, l'épaisseur du fond de PEEK est choisie comprise entre 0.3 mm et 0.8 mm, préférentiellement comprise entre 0.3mm et 0.6 mm, et encore plus préférentiellement sensiblement égale à 0,4 mm ($\pm$ 0.05 mm).

L'invention propose un appareil de traitement d'une pathologie comprenant :

- un dispositif ultrasonore comportant une carte électronique et au moins un transducteur connecté électriquement à la carte électronique, le transducteur étant apte à générer des ondes ultrasonores,
- une unité de commande distante pour déterminer et contrôler des paramètres de fonctionnement du dispositif ultrasonore, et lui délivrer de l'électricité pendant au moins un cycle de traitement, chaque cycle de traitement étant précédé d'un cycle d'attente,
- des moyens de connexion électrique entre le dispositif ultrasonore et l'unité de commande,

**remarquable en ce que** l'unité de commande est programmée pour mettre en œuvre une phase de détection d'un défaut de fonctionnement de chaque transducteur du dispositif ultrasonore, ladite phase de détection comprenant :

- l'émission par l'unité de commande, d'au moins un signal de test, chaque signal de contrôle ayant un fréquence nulle,
- la mesure par l'unité de commande, d'au moins un signal réfléchi de test,
- le traitement du signal réfléchi de test pour détecter :

  ◦ soit un court-circuit dans le dispositif ultrasonore,
  ◦ soit un défaut de connexion électrique entre ledit et au moins un transducteur et la carte électronique.

## BREVE DESCRIPTION DES DESSINS

D'autres avantages et caractéristiques du procédé et du système selon l'invention ressortiront mieux de la description qui va suivre de plusieurs variantes d'exécution, données à titre d'exemples non limitatifs, à partir des dessins annexés sur lesquels :

- la figure 1 illustre schématiquement un exemple d'appareil de traitement d'une affection cérébrale incluant un dispositif ultrasonore raccordé électriquement à une unité de commande distante grâce à des moyens de connexion (aiguille transdermique + câble),

- la figure 2 est une représentation schématique en coupe d'un des transducteurs du dispositif ultrasonore,
- la figure 3 illustre des spectres d'absorption de la puissance électrique des transducteurs de thérapie,
- la figure 4 est une représentation schématique des principales étapes d'un procédé d'estimation de la qualité d'un couplage acoustique,
- la figure 5 est une courbe illustrant, pour un lot de transducteurs, la puissance consommée par chaque transducteur en fonction de la fréquence du signal électrique appliqué,
- la figure 6 est une représentation schématique d'une variante de réalisation du procédé d'estimation de la qualité d'un couplage acoustique
- la figure 7 est une représentation schématique d'une variante de réalisation d'un procédé de détection d'un défaut de fonctionnement d'un transducteur.

## DESCRIPTION DETAILLEE DE L'INVENTION

On va maintenant décrire différents exemples du système et du procédé pour l'estimation de la qualité d'un couplage acoustique en référence aux figures. Dans ces différentes figures, les éléments équivalents sont désignés par la même référence numérique.

Ce système et ce procédé d'estimation permettent à un praticien de vérifier si le couplage acoustique entre un dispositif ultrasonore implanté dans le corps d'un patient et un tissu à traiter est correctement réalisé.

Dans la suite, on décrira le système et le procédé d'estimation en référence à l'appareil présenté dans le document EP 2 539 021 auquel la demande internationale WO 2018/007500 fait référence.

Toutefois, il est bien évident pour l'homme du métier que le système et le procédé selon l'invention peuvent être mis en œuvre avec tout type d'appareil de traitement incluant un dispositif implantable ou non-implantable devant être couplé acoustiquement à un tissu à traiter.

### 1. *Généralités*

### 1.1. *Appareil de traitement*

Comme décrit précédemment, l'appareil comprend :

- un dispositif ultrasonore 1,
- une unité de commande 2, et
- des moyens de connexion.

Le dispositif ultrasonore 1 est destinée à être implantée dans un os crânien d'un patient. Il comporte :

- une carte électronique adaptée pour échanger des signaux électriques d'alimentation et de contrôle avec l'unité de commande distante,
- un transducteur 12 connecté à la carte électronique pour la génération d'ondes ultrasonores, et
- une borne de connexion 14 destinée à recevoir une aiguille transdermique 32 des moyens de connexion électrique.

Dans la suite, on considèrera que le dispositif ultrasonore 1 a été implanté, c'est-à-dire que :

- le dispositif ultrasonore 1 a été inséré dans une ouverture crânienne de sorte que le (ou les) transducteur(s) s'étende(nt) en regard d'un tissu à traiter, que
- le dispositif ultrasonore 1 a été fixé sur la périphérie de l'ouverture crânienne par tout moyen connu de l'homme du métier (vis d'ancrage, collage, etc.), puis que
- le cuir chevelu et les muscles de la tête du patient ont été remis en place pour recouvrir le dispositif ultrasonore 1.

L'unité de commande 2 distante permet d'alimenter en énergie électrique le dispositif ultrasonore 1, de régler ses paramètres de fonctionnement et de recevoir un signal de réfléchie par le dispositif. Une telle unité de commande 2 étant connue de l'homme du métier, celle-ci ne sera pas décrite plus en détails dans la suite.

Les moyens de connexion permettent de raccorder électriquement le dispositif ultrasonore 1 et l'unité de commande 2. Les moyens de connexion comportent notamment :

- l'aiguille transdermique 32 apte à être branchée sur la borne de connexion du dispositif ultrasonore,

- un câble électriquement conducteur 31, et
- une prise de liaison (non représentée) apte à être branchée à une prise complémentaire de l'unité de commande 2.

### 1.2. *Principe d'utilisation de l'appareil*

Un tel appareil permet le traitement d'une affection cérébrale en mettant en œuvre plusieurs séances de traitement prescrites par le praticien.

A chaque nouvelle séance de traitement, le praticien raccorde électriquement le dispositif ultrasonore 1 à l'unité de commande 2 distante en utilisant les moyens de connexion.

Plus précisément, le praticien connecte la prise de liaison à l'unité de commande 2 distante. Le praticien insère ensuite l'aiguille transdermique 32 dans le cuir chevelu du patient, et introduit l'extrémité de l'aiguille 32 dans un trou borgne de la borne de connexion 14 de sorte à finaliser le raccordement électrique du dispositif ultrasonore 1 à l'unité de commande 2 distante.

Une fois le dispositif ultrasonore 1 raccordé à l'unité de commande 2, une succession de cycles de traitement sont exécutés, chaque cycle de traitement étant précédé d'un cycle d'attente.

Durant un cycle d'attente, le dispositif ultrasonore 1 est désactivé pendant une durée d'attente (de l'ordre de 1 seconde). Cette désactivation est réalisée en n'alimentant pas le dispositif ultrasonore 1 en énergie électrique.

Lorsque la durée d'attente est expirée, un cycle de traitement est mis en œuvre. Durant le cycle de traitement, le dispositif ultrasonore 1 est alimenté en énergie électrique par l'application, au niveau de la borne de connexion, d'un signal électrique d'excitation pendant une durée de traitement (de l'ordre de 25 millisecondes).

Ce signal électrique d'excitation est émis par l'unité de commande 2 à une fréquence de travail du (ou des) transducteur(s) 12.

On entend, dans le cadre de la présente invention, par *« fréquence de travail »* (ou *« fréquence de traitement »*) la fréquence d'émission des ondes ultrasonores de traitement émises par le (ou les) transducteur(s) 12, cette fréquence correspondant également à la fréquence du signal électrique d'excitation permettant d'alimenter le dispositif ultrasonore en énergie électrique.

Cette fréquence de travail est contenue dans une bande utile de fréquences du transducteur 12 (i.e. plage de fréquences de fonctionnement du transducteur), le transducteur 12 ne fonctionnant pas (i.e. ne générant pas d'ondes ultrasonores) lorsqu'un signal électrique de fréquence non-contenue dans cette bande utile lui est appliqué.

Avantageusement, les transducteurs 12 peuvent être choisis pour avoir un rendement maximal à la fréquence de travail. Ainsi, la fréquence de travail correspond à une fréquence de traitement des ondes ultrasonores utilisées pour traiter le tissu à traiter.

En réponse à l'application du signal électrique d'excitation pendant la durée de traitement, le transducteur 12 génère des ondes ultrasonores en direction du tissu à traiter.

Lorsque la durée de traitement est expirée, un nouveau cycle d'attente est mis en œuvre, et ainsi de suite jusqu'à la fin de la séance.

### 2. *Transducteur*

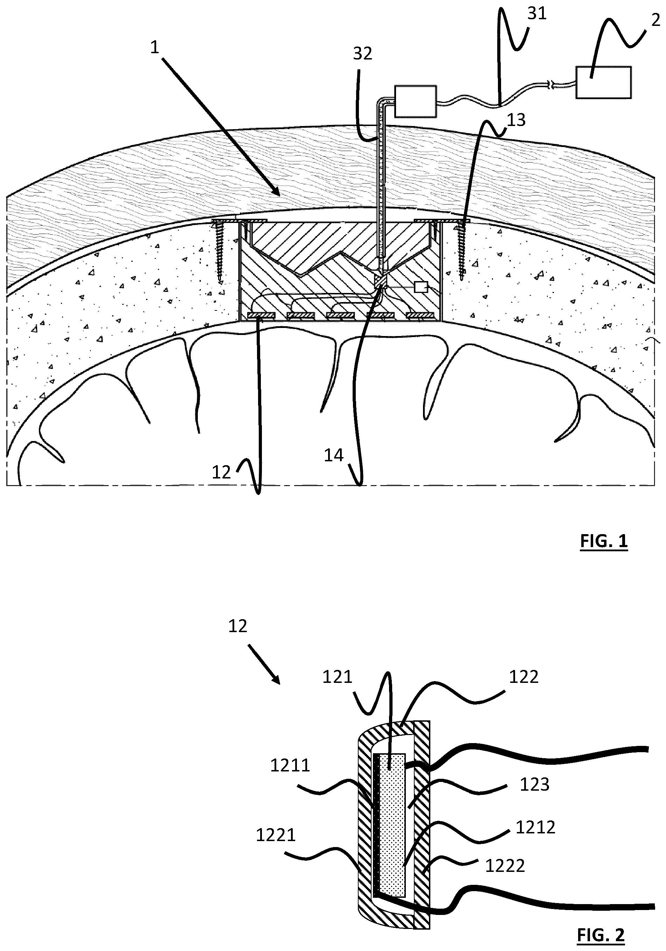

En référence à la figure 2, on a illustré une vue en coupe partielle d'un des transducteurs 12 du dispositif ultrasonore 1.

Le transducteur 12 comporte :

- un (ou plusieurs) élément électro-acoustiques 121 destiné(s) à la thérapie (les trains d'impulsion sont longs et à fréquence fixe), et
- un boitier 122 contenant l'élément électro-acoustique.

### 2.1. *Elément électro-acoustique(s)*

Chaque élément électro-acoustique de thérapie 121 est constitué dans un matériau piézo-électrique, tel que du *« composite »* (association d'au moins un matériau piézo-électrique avec un ou plusieurs matériaux non piézo-électriques tel qu'un polymère, etc.).

Lorsque l'élément piézoélectrique 121 est du type *« composite »* son impédance acoustique est proche de celle du tissu et une lame quart d'onde n'est pas nécessaire, en particulier lorsque le dispositif est destiné à la thérapie.

Chaque élément électro-acoustique 121 est fixé sur un fond 1221 du boitier 122, par exemple par collage en utilisant une fine couche de colle (qui joue un rôle acoustique négligeable à la fréquence de travail du transducteur).

Comme illustré à la figure 2, le transducteur 12 comprend également en face arrière 1212 de l'élément électro-acoustique 121 une couche réfléchissante (ou *« backing »* selon la terminologie anglo-saxonne), tel qu'une (ou plusieurs) couche(s) d'air 123, la (ou chaque) couche d'air 123 s'étendant sur la face arrière 1212 de l'élément électro-acoustique 121.

Ainsi, le transducteur 12 est dépourvu de matériau absorbant en face arrière 1212 de l'élément électro-acoustique 121, contrairement aux dispositifs d'imagerie acoustique (utilisant la technique dite de *« pulse-écho »*) dans lesquels la face arrière de chaque élément électro-acoustique est recouverte d'un matériau absorbant pour éviter que l'élément ne résonne pendant une durée importante postérieurement à son excitation.

Enfin, un transducteur de thérapie émet des énergies importantes (du fait notamment de la durée des émissions) et ne doit donc pas monter en température, surtout s'il est implanté chez un patient.

La présence de matériau absorbant n'est donc pas souhaitable en face arrière du (ou des) élément électro-acoustique (s) 121 du (ou des) transducteur 12.

Le lecteur appréciera également que la couche d'air disposée en face arrière du (ou des) élément(s) électro-acoustique(s) 121 permet d'améliorer le rendement énergétique du transducteur en réfléchissant toute l'énergie acoustique générée par l'élément vers la face avant de celui-ci.

En effet, l'élément piézoélectrique 121 comprend :

- une face avant 1211 dirigée vers le tissu à traiter, et
- une face arrière 1212 opposée à la face avant 1211.

Lorsque l'élément 121 est alimenté en énergie électrique, celui-ci convertit l'énergie électrique en énergie mécanique et sa vibration génère une onde acoustique qui peut se propager vers l'avant et vers l'arrière de l'élément.

Une couche d'air 123 en face arrière 1212 de l'élément piézoélectrique agit comme un miroir et réfléchit l'onde dirigée vers l'arrière de l'élément 121 en direction de sa face avant 1211. Ainsi, on évite de perdre une partie de l'énergie mécanique générée par l'élément 121.

### 2.2. Boitier

Le boîtier 122 comprend le fond 1221, une paroi latérale et un couvercle 1222. Avantageusement, le matériau constituant le boîtier 122 peut être du Poly-Ether-Ether-Ketone (ci-après dénommé « *PEEK* »). Le PEEK est particulièrement adapté à la fabrication d'un dispositif implantable du fait de ses nombreuses qualités. Le PEEK est en effet un matériau :

- fortement étanche,
- biocompatible,
- isolant électrique, et
- stable dans le temps (lorsqu'immergé).

Dans le mode de réalisation illustré à la figure 2, pour une fréquence de travail de l'ordre de 1MHz, l'épaisseur du fond 1221 de PEEK (en regard de la face avant du (ou de chaque) élément 121) est choisie comprise entre 0.3 mm et 0.8 mm, préférentiellement comprise entre 0.3mm et 0.6 mm, et encore plus préférentiellement sensiblement égale à 0,4 mm (± 0.05 mm).

Bien entendu, le choix de l'épaisseur du fond est fonction de la fréquence de travail utilisée pour le transducteur 12. Ainsi, le choix de l'épaisseur en fonction de la fréquence de travail satisfait la relation suivante :

$$E_{Fond} = (V_{son} / 4F_{Travail}) \times (0.8 \pm 0.4) \, ,$$

Où :

- $E_{Fond}$ correspond à l'épaisseur du fond du boitier (en mm),
- $V_{son}$ correspond à la vitesse du son dans le matériau constituant le fond du boitier, et
- $F_{Travail}$ correspond à la fréquence de travail du transducteur 12 (en MHz), ladite fréquence de travail étant choisie dans la bande de fréquence utile du transducteur 12.

Un tel choix d'épaisseur pour le fond 1221 du boitier 122 va à l'encontre des connaissances générales de l'homme du métier qui choisirait une épaisseur de fond la plus fine possible afin :

- de limiter l'absorption par le fond du boitier 122, de l'énergie ultrasonore émise par le (ou les) éléments(s) 121, et
- de réduire le volume du dispositif ultrasonore implantable.

Au contraire, ce choix d'épaisseur de fond 1221 (dans le cas d'un fond en PEEK) est effectué pour faciliter la détection d'un mauvais couplage acoustique entre le dispositif ultrasonore 1 et le tissu à traiter.

En effet l'utilisation d'un fond 1221 en PEEK d'épaisseur sensiblement égale à 0,4 mm ($\pm$ 0.05 mm) permet de faciliter la détection d'une bulle de gaz entre le transducteur 12 et le tissu à traiter, les spectres d'absorption électrique étant très différents selon que le fond est en contact acoustique avec un gaz d'une part, ou avec le milieu de propagation (dans le cas d'espèce : la dure-mère) d'autre part.

Un transducteur 12 peut comporter plusieurs éléments piézo-électriques 121 montés dans un même boîtier 122. Chaque boitier est étanche.

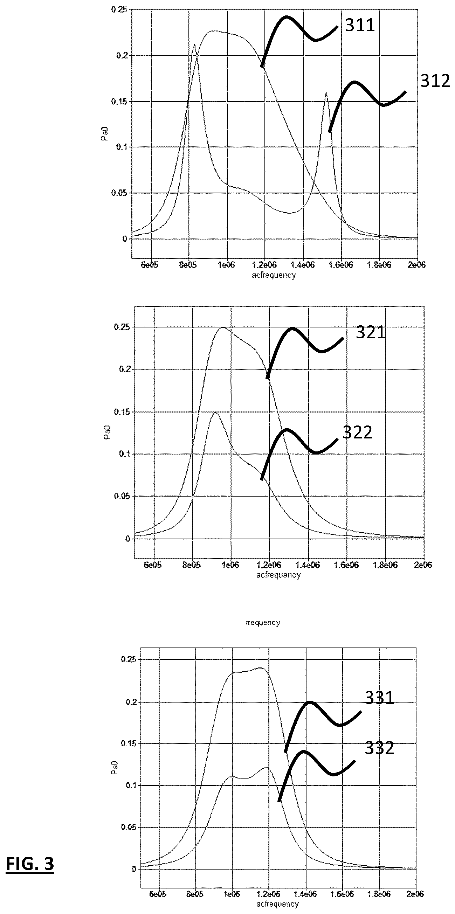

A titre indicatif, la figure 3 illustre des spectres d'absorption en puissance électrique (puissance active) de transducteurs associés à des fonds de boitier 1221 d'épaisseurs différentes. L'ordonnée $P_{a0}$ des graphiques correspond à la puissance absorbée par le transducteur pour une puissance incidente de 250mW. Noter qu'une adaptation d'impédance électrique a été insérée entre le transducteur et le générateur :

- le premier spectre d'absorption (référencé 311 dans le cas d'un couplage acoustique avec le milieu de propagation, et référencé 312 dans le cas d'un couplage acoustique avec un gaz) correspond à un transducteur associé à un fond de PEEK d'épaisseur égale à 0,4 mm,
- le deuxième spectre d'absorption (référencé 321 dans le cas d'un couplage acoustique avec le milieu de propagation, et référencé 322 dans le cas d'un couplage acoustique avec un gaz) correspond à un transducteur associé à un fond de PEEK d'épaisseur égale à 0,2 mm,
- le troisième spectre d'absorption (référencé 331 dans le cas d'un couplage acoustique avec le milieu de propagation, et référencé 332 dans le cas d'un couplage acoustique avec un gaz) correspond à un transducteur associé à un fond de PEEK d'épaisseur nulle, c'est-à-dire un boitier dépourvu de fond (la face avant du transducteur étant uniquement recouverte d'une couche de parylène).

Comme il ressort clairement de cette figure 3, avec une épaisseur de PEEK de 400 $\mu$m, il est possible de mettre à profit des émissions à fréquences différentes pour vérifier le couplage acoustique entre le transducteur 12 et le tissu à traiter.

Ainsi pour une fréquence de travail de l'ordre de 1MHz, le choix d'un boitier 122 en PEEK dont le fond présente une épaisseur comprise entre 0.3 mm et 0.8 mm, préférentiellement comprise entre 0.3mm et 0.6 mm, et encore plus préférentiellement sensiblement égale à 0,4 mm ($\pm$ 0.05 mm) permet :

- d'augmenter la bande passante pour la thérapie (pouvoir utiliser une gamme de fréquence plus large, être plus robuste à la variabilité), sans diminuer le rendement électroacoustique,
- d'augmenter l'isolation électrique, la biocompatibilité, et la longévité du dispositif ultrasonore ; en effet, il est connu que les céramiques PZT ((Titano-Zirconate de Plomb) ne sont pas biocompatible (présence de plomb), que le parylène (pouvant être utilisé en tant que film de protection pour recouvrir chaque transducteur) est poreux, et que les matériaux de type PZT ou « composite » dérivent avec l'humidité dans la durée.

Plus généralement, il est possible de comparer les spectres de puissance réfléchie (ou mesures d'impédance électrique) à un modèle de référence ou gabarit (composé d'une courbe de valeur de référence minimale et d'une courbe de valeur de référence maximale). Si la courbe représentative des spectres de puissance réfléchie mesurée n'est pas contenue dans le gabarit, ceci est représentatif d'un défaut. En regardant pour quelle fréquence le spectre de puissance réfléchie est en dehors du gabarit, il est possible de définir ce défaut (air, connexion, court-circuit, transducteur défaillant, etc.).

On entend par *« puissance incidente »* la puissance transmise vers le transducteur 12 par l'unité de commande 2. On entend par *« puissance active »,* la puissance consommée par le transducteur 12 (puissance incidente - puissance réfléchie : une partie est convertie en chaleur et l'autre en ultrasons). On entend par *« puissance réfléchie »* la puissance circulant du transducteur 12 vers l'unité de commande 2.

On définit de même le signal électrique réfléchi ($\phi_r$) et le signal électrique incident ($\phi_0$) comme les amplitudes des ondes électriques réfléchies et incidentes.

Le lecteur appréciera qu'il est possible d'acquérir les spectres de puissance active /puissance réfléchie /impédance selon plusieurs méthodes, par exemple :

- De manière continue (par exemple sur la gamme 0.2- 1.6 MHz) en émettant un signal test modulé en fréquence de type *« chirp »,* ou un signal très court et procéder à une analyse de Fourier,
- Ou de manière discrète, (ce qui permet de limiter le coût de l'électronique associée) en émettant plusieurs impulsions de tests à des fréquences différentes judicieusement choisies pour permettre de détecter plusieurs types de défauts à partir d'un nombre limité d'impulsions de fréquences différentes (par exemple quatre impulsions à quatre fréquences différentes).

### 2.3. *Avantages associés à la configuration décrite ci-dessus*

La configuration du transducteur décrite ci-dessus (couche réfléchissante en face arrière de l'élément piézo-électrique et couche de PEEK en face avant de l'élément piézo-électrique) permet d'augmenter la capacité de discrimination entre la présence d'air et la présence d'eau au niveau de la face avant du transducteur, c'est à dire la vérification du bon couplage lorsque le transducteur est implanté. Comme le transducteur (air + piézo-composite + ¼ d'onde) est très bien adapté à l'eau et n'est pas amorti au niveau de sa face arrière, les ondes ultrasonores ne peuvent être amorties que par la face avant (couplage eau/cerveau). Ainsi, la présence d'eau ou non en face avant a une très grande influence sur l'impédance électrique du transducteur.

La configuration du transducteur permet également :

- d'améliorer la bande passante du transducteur sans diminuer le rendement électroacoustique, ce qui permet plus de souplesse d'utilisation ;
- d'augmenter l'isolation électrique, la biocompatibilité, et la longévité du transducteur.

### 3. *Problématique associée à l'utilisation de l'appareil de traitement*

Comme indiqué précédemment, la qualité du couplage acoustique (entre le dispositif ultrasonore et le milieu contenant le tissu à traiter) peut varier au cours du temps.

Par exemple au cours d'une séance, une bulle de gaz peut se former entre le (ou l'un des) transducteur(s) 12 et le tissu à traiter. De même, une bulle de gaz peut être emprisonnée entre le transducteur et le tissu à traiter lors de l'opération d'implantation du dispositif. Également, une calcification osseuse peut se former au court du temps entre le transducteur et le tissu à traiter. La présence d'un tel matériau réfléchissant (bulle de gaz ou croissance osseuse) entre le transducteur et le tissu limite la propagation des ondes ultrasonores générées par le transducteur 12 vers le tissu à traiter, ce qui a pour conséquence de limiter l'efficacité du traitement.

De plus, du liquide peut pénétrer dans le dispositif 1, par exemple lors de l'insertion de l'aiguille transdermique 32 dans la borne de connexion 14, ce liquide pouvant provoquer un court-circuit (ou plus précisément l'apparition d'un courant de fuite) limitant l'efficacité du traitement.

C'est pourquoi il est souhaitable d'estimer la qualité du couplage acoustique entre le dispositif ultrasonore 1 et le tissu à traiter afin de limiter les risques d'inefficacité du traitement.

Par ailleurs, un (ou plusieurs) des transducteurs peut (peuvent) présenter un défaut tel que court-circuit ou circuit ouvert (par exemple suite à la désolidarisation d'une (ou de plusieurs) patte(s) de connexion d'un (ou de plusieurs) transducteur(s)).

C'est pourquoi il est également souhaitable de détecter un défaut de fonctionnement d'un (ou de plusieurs) transducteur(s) du dispositif ultrasonore afin de limiter les risques d'inefficacité du traitement.

Le lecteur appréciera que ces deux phases de test (i.e. estimation de la qualité du couplage et détection d'un défaut de fonctionnement d'un transducteur) peuvent être réalisés indépendamment l'un de l'autre, ou conjointement. Ainsi dans certains modes de réalisation, l'appareil de traitement peut être configuré pour :

- réaliser uniquement une estimation de la qualité du couplage acoustique, ou
- réaliser uniquement une détection d'un défaut de fonctionnement d'un (ou de plusieurs) transducteur(s) du dispositif ultrasonore, ou
- réaliser à la fois une estimation de la qualité du couplage acoustique et une détection d'un défaut de fonctionnement d'un (ou de plusieurs) transducteur(s) du dispositif ultrasonore.

### 4. *Procédé d'évaluation de la qualité du couplage acoustique*

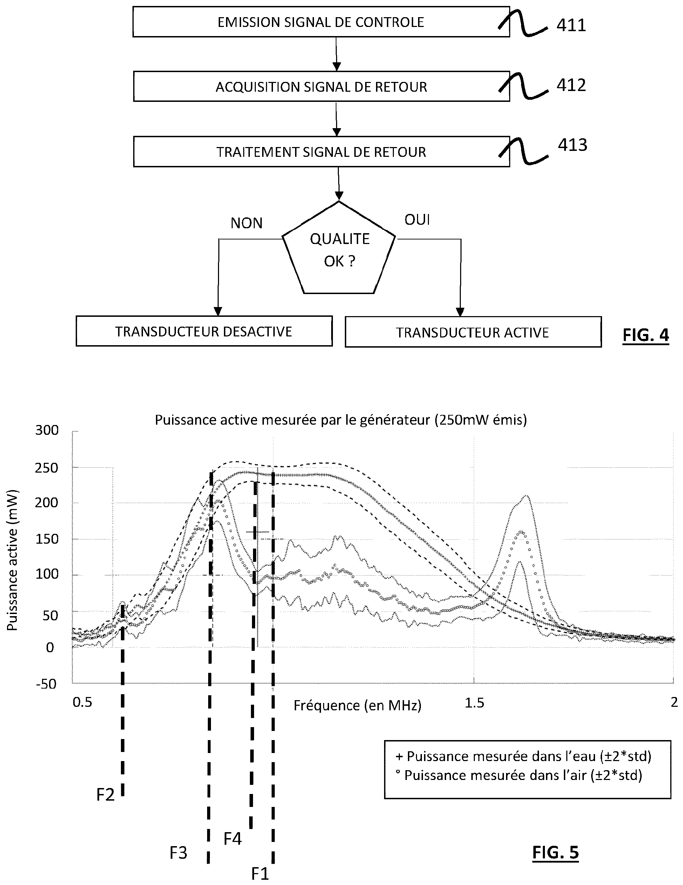

En référence à la figure 4, le procédé d'estimation de la qualité du couplage acoustique comprend les étapes suivantes :

- l'émission 411 par l'unité de commande 2 d'au moins un signal de contrôle,
- l'acquisition 412 par l'unité de commande 2 d'au moins un signal de retour,
- le traitement 413 du signal de retour pour obtenir une information sur la qualité du couplage acoustique entre le dispositif ultrasonore 1 et le tissu à traiter, ledit traitement permettant :

    ▪ l'activation de chaque transducteur 12 pour lequel la qualité du couplage acoustique est supérieure à un seuil de qualité,

- la désactivation de chaque transducteur 12 pour lequel la qualité du couplage acoustique est inférieure à un seuil de qualité.

Suite à l'évaluation de la qualité du couplage acoustique, les transducteurs activés peuvent (pendant chaque cycle de traitement) être alimentés en énergie électrique de sorte qu'ils génèrent des ondes ultrasonores de traitement vers le tissu à traiter. Les transducteurs désactivés ne sont quant à eux pas alimentés en énergie électrique par le dispositif de commande 2.

Chaque signal de contrôle est émis à une faible énergie électrique par rapport au signal d'excitation (de l'ordre de 1% de l'énergie requise pour le traitement). Plus précisément, la puissance électrique de chaque signal de contrôle est telle que les éventuelles ondes ultrasonores générées par le dispositif ultrasonore (en réponse au signal de contrôle) ne provoquent aucun effet tissulaire.

Afin de détecter la présence éventuelle de l'un de ces facteurs (i.e. de fluide dans le dispositif ultrasonore et/ou bulle de gaz/os entre le fond du boitier et le milieu de propagation, et/ou défaut de fonctionnement du transducteur), plusieurs signaux de contrôle sont émis chacun à une fréquence. Pour chaque signal, l'unité de commande 2 émet un signal d'amplitude et de fréquence connues vers le dispositif 1. Celui-ci n'étant pas parfaitement adapté en impédance notamment en raison d'une adaptation acoustique imparfaite entre le transducteur 12 et le tissu - une partie du signal (signal de retour) est réfléchie vers le dispositif. L'unité de commande 2 mesure l'amplitude de ce signal réfléchi et en déduit un taux de réflexion. L'unité de commande 2 peut aussi mesurer l'impédance du circuit du dispositif ultrasonore implanté jusqu'au transducteur.

Plus précisément, le procédé comprend les étapes suivantes pour chaque transducteur 12 du dispositif ultrasonore 1 :

- application de plusieurs signaux de contrôle à plusieurs fréquences, chaque signal de contrôle ayant une fréquence respective,
- mesure d'une pluralité de taux de réflexion des signaux de contrôle, chaque taux de réflexion mesuré correspondant à un signal de contrôle respectif,
- comparaison des taux de réflexion avec des seuils préétablis et estimation de la qualité du couplage entre le transducteur 12 considéré et le tissu.

Le taux de réflexion correspond à la proportion de signal de contrôle réfléchie par le transducteur 12. Le taux de réflexion (B) d'un signal de contrôle peut être défini comme le rapport entre le signal électrique réfléchi ($\phi_r$) et le signal électrique incident ($\phi_0$) :

$$B = \phi_r \, / \, \phi_0.$$

En pratique, l'étape d'application des signaux de contrôle consiste à appliquer séquentiellement deux, trois ou quatre signaux de contrôle ayant chacun une fréquence respective.

Les fréquences des signaux de contrôle sont choisies pour maximiser la discrimination (de sensibilité de détection de problème) pour chacun des quatre facteurs suivants :

- Défaut de raccordement électrique tel que proposé dans WO 2018/007500,
- Présence d'une bulle de gaz entre le transducteur 12 et le tissu à traiter,
- Présence de fluide dans le dispositif ultrasonore (par exemple au niveau de la borne de connexion contenant l'extrémité de l'aiguille transdermique 32),
- Défaut de fonctionnement d'un transducteur (court-circuit ou circuit ouvert)

Le choix des fréquences se fait en fonction :

- des spectres d'absorption et
- des écarts-types des spectres d'absorption

dans une population des transducteurs fabriqués.

Notamment, les fréquences des signaux de contrôle sont choisies telles que le rapport suivant soit maximal :

$$(\text{Moyenne}_{eau} - K\sigma_{eau}) \, / \, (\text{moyenne}_{air} + K\sigma_{air}),$$

Avec :

- « *Moyenne »,* la moyenne des mesures d'absorption sur une population d'échantillons à une fréquence considérée,
- « $\sigma$ », l'écart type des mesures d'absorption sur la population d'échantillons à la fréquence considérée (la légère variabilité de fonctionnement des transducteurs dues aux tolérances de fabrication expliquant la présence de cet écart type),
- « *K* » un entier compris entre 1 et 3.

L'avantage de l'utilisation de différents signaux de contrôle ayant chacun une fréquence respective (associé au choix d'épaisseur de fond du boitier) est de rendre le procédé d'évaluation très discriminant vis-à-vis des différents facteurs susceptibles de détériorer la qualité du couplage. En d'autres termes, le procédé selon l'invention permet de définir si une qualité de couplage insuffisante est due :

- à un défaut de raccordement,
- à la présence d'une bulle de gaz, ou
- à la présence de fluide.
- à un défaut de fonctionnement d'un des transducteurs

Il est ainsi possible d'informer plus précisément le praticien sur la nature du problème détecté afin qu'il puisse mettre en œuvre la solution la plus adaptée pour résoudre le problème détecté.

*4.1. Fréquences*

Pour déterminer d'éventuels défauts de couplage rendant le traitement impossible ou inefficace, différents signaux de contrôle à différentes fréquences sont émis, chaque signal de contrôle ayant une fréquence (de préférence) différente de la fréquence de traitement $F_1$.

*4.1.1. Fréquence du signal de contrôle pour la détection d'un court-circuit*

En particulier le signal de contrôle pour la détection d'un court-circuit ou d'une résistance parasite (dû à la présence de liquide au niveau de la borne de connexion) est émis à une fréquence $F_2$.
Cette fréquence $F_2$ est choisie très inférieure à la fréquence de travail $F_1$ telle que la puissance consommée par le transducteur soit faible (inférieure à 40% de la puissance incidente).
Plus précisément, la fréquence $F_2$ est choisie en dehors de la bande utile de fréquences du transducteur (i.e. plage de fréquences de fonctionnement du transducteur) ; ainsi un taux de réflexion mesuré (à la fréquence $F_2$) très inférieur à 1 (i.e. puissance consommée non nulle) indique l'existence d'un court-circuit dû à la présence de liquide dans le dispositif ultrasonore.
Notamment, dans un mode de réalisation de l'invention, la fréquence $F_2$ du signal de contrôle utilisé pour la détection d'un court-circuit ou d'une résistance parasite est sensiblement égale à 0,6 MHz.

*4.1.2. Fréquence du signal de contrôle pour la détection d'un défaut de raccordement électrique*

Le signal de contrôle pour la détection d'un défaut de raccordement électrique (voir WO2018007500) est émis à une fréquence $F_3$, différente de la fréquence $F_2$.
Cette fréquence $F_3$ est choisie comprise entre la fréquence $F_2$ et la fréquence de travail $F_1$. Notamment, dans un mode de réalisation de l'invention, la fréquence $F_3$ du signal de contrôle utilisé pour la détection d'un défaut de raccordement électrique est sensiblement égale à 850 kHz.
**En particulier,** la fréquence $F_3$ est choisie telle que la puissance consommée par le transducteur soit indépendante du milieu situé en face avant du transducteur. En d'autres termes, la fréquence $F_3$ est choisie telle que :

- la puissance consommée par le transducteur soit maximale (notamment supérieure à 40% de la puissance incidente du signal de contrôle appliqué au transducteur) d'une part, et que
- la puissance consommée par le transducteur lorsque sa face avant est en contact avec un gaz soit sensiblement égale à la puissance consommée par le transducteur lorsque sa face avant est en contact avec un liquide ou le tissu.

Ainsi un taux de réflexion mesuré (à la fréquence $F_3$) sensiblement égale à 1 (i.e. puissance active (=consommée) nulle) indique l'absence de connexion électrique entre le dispositif ultrasonore 1 (ou l'un des transducteurs 12 du dispositif 1) et l'unité de commande 2.

### 4.1.3. *Fréquence du signal de contrôle pour la détection d'une bulle de gaz*

Le signal de contrôle pour la détection d'une bulle de gaz entre le transducteur et le tissu à traiter est émis à une fréquence $F_4$ différente des fréquences $F_2$ et $F_3$.

En particulier, la fréquence $F_4$ est choisie telle que la puissance consommée par le transducteur soit :

- à un minimum lorsque la face avant du transducteur est en regard d'une bulle de gaz (un transducteur est considéré dans l'air si la puissance transmise est inférieure à 64% de la puissance incidente, et dans l'eau dans le cas contraire), et

- à un maximum lorsque la face avant du transducteur est en regard d'un milieu de propagation tel que la dure-mère, le tissu ou du liquide.

Cette fréquence $F_4$ est choisie supérieure à la fréquence $F_3$ et légèrement inférieure (i.e. entre 1% et 10% inférieure, préférentiellement 1% et 5% inférieure) ou égale à la fréquence de travail $F_1$.

Notamment, dans un mode de réalisation de l'invention, la fréquence $F_4$ du signal de contrôle utilisé pour la détection d'une bulle de gaz est sensiblement égale à 960 kHz (96% de la fréquence de travail du transducteur).

Ainsi et comme illustré à la figure 5 (qui représente la puissance consommée par un lot de transducteurs en fonction de la fréquence du signal électrique appliqué de puissance 250mW), les fréquences utilisées pour les différents signaux de contrôle sont choisies de sorte à maximiser la discrimination entre les différents types de défauts susceptibles d'influer sur la qualité du traitement.

### 4.1.4 *Fréquence du signal de contrôle pour la détection d'un défaut de fonctionnement d'un transducteur (tension continue : $F_0=0$)*

Le signal de contrôle pour la détection d'un défaut de fonctionnement d'un transducteur est émis à une fréquence $F_0$ nulle de sorte que le signal de contrôle présente une tension continue.

Ainsi, un signal de contrôle à tension continue peut être appliquée par l'unité de commande au dispositif ultrasonore implanté. Ce signal de contrôle à fréquence nulle $F_0$ permet de détecter soit :

- un court-circuit franc dans un transducteur (impédance nulle, quand le transducteur est activée)
  b) un défaut dans une chambre de connexion (impédance trop faible si présence de liquide, notamment si l'impédance est trop faible qu'un transducteur soit commandé ou non ou quel que soit le transducteur commandé).

Ce test permet de compléter le test décrit au point 4.1.1. pour la détection d'un court-circuit à partir d'un signal de contrôle émis à la fréquence $F_2$.

### 4.2. *Exemple de mise en œuvre du procédé d'estimation*

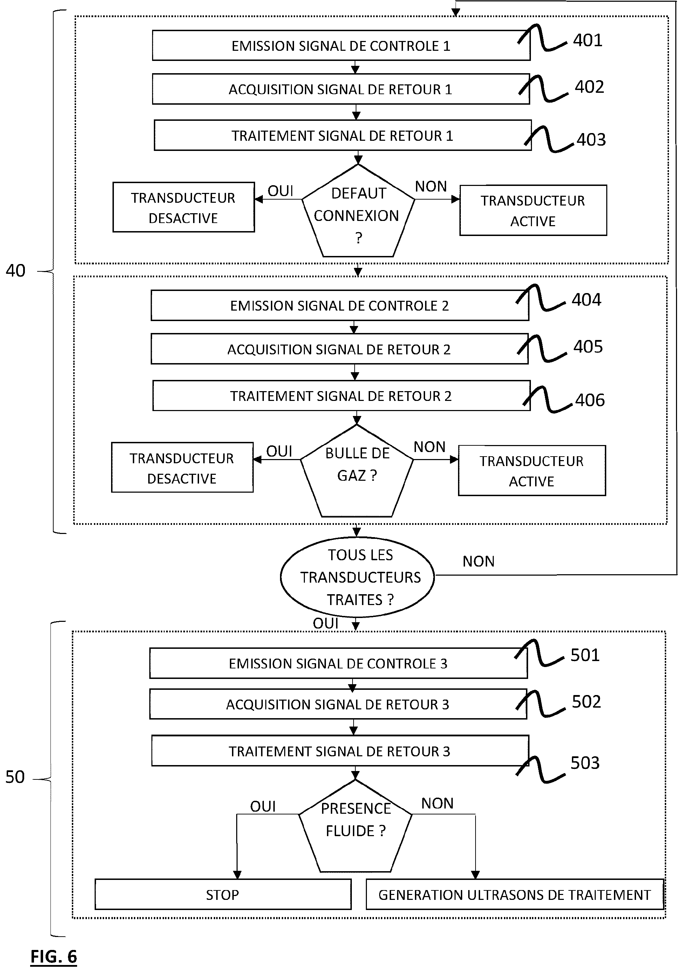

On va maintenant décrire plus en détails le principe de fonctionnement du procédé d'estimation en référence à la figure 6. Ce procédé d'estimation permet de détecter :

- la présence d'air en face avant des émetteurs,
- si les émetteurs sont fonctionnels,
- si un fluide (tel que de l'eau) est présent à l'intérieur du dispositif ultrasonore.

**Dans ce** mode de réalisation, certaines étapes de détection du procédé réalisées pendant chaque cycle d'attente, et d'autres réalisées pendant chaque cycle de traitement.

### 4.2.1. *Cycle d'attente*

Pendant chaque cycle d'attente, le procédé comprend :

- une première étape de détection d'un défaut de raccordement électrique, et
- une deuxième étape de détection de la présence d'une bulle de gaz.

Ces première et deuxième étapes sont mises en œuvre séquentiellement, pour chaque transducteur 12 du dispositif ultrasonore 1.

#### 4.2.1.1. *Défaut de raccordement électrique*

La première étape de détection d'un défaut de raccordement électrique comporte les sous-étapes consistant à :

- Emettre 401 un premier signal de contrôle à une première fréquence de contrôle $F_3$:

  ○ le premier signal de contrôle consiste par exemple en un signal impulsionnel de puissance 250mW et de durée égale à 1ms,
  ○ la première fréquence étant choisie égale à 850 kHz,

- Acquérir 402 un premier signal réfléchi de contrôle correspondant à la portion du premier signal de contrôle n'ayant pas été absorbée par le dispositif ultrasonore :

  o l'acquisition du premier signal réfléchi peut consister à mesurer la puissance électrique du signal réfléchi (par exemple en utilisant un coupleur directionnel) ou toute autre information représentative de la puissance consommée par le (ou les) transducteur(s),

- Traiter 403 le premier signal réfléchi de contrôle pour détecter un défaut de raccordement :

  ○ durant la sous-étape consistant à traiter, une information représentative de la puissance consommée par le transducteur est extraite du premier signal réfléchi,
  ○ **cette** information représentative de la puissance consommée est comparée à un premier seuil prédéfini correspondant à 40% de la puissance du premier signal de contrôle (soit 100mW dans le cas d'un premier signal de contrôle de 250mW) :

    ▪ si l'information représentative de la puissance consommée est inférieure au premier seuil, alors le transducteur 12 n'est pas correctement raccordé à l'unité de commande distante 2 (i.e. détection d'une absence de liaison électrique entre le transducteur et l'unité de commande),
    ▪ sinon, alors le raccordement électrique entre le transducteur considéré et l'unité de commande est fonctionnel (i.e. aucune imperfection dans la connexion électrique entre le transducteur et l'unité de commande).

#### 4.2.1.2. *Présence de gaz*

L'étape de détection de la présence d'une bulle de gaz comporte les sous-étapes consistant à :

- Emettre 404 un deuxième signal de contrôle à une deuxième fréquence de contrôle :

  ○ le deuxième signal de contrôle consiste par exemple en un signal impulsionnel de puissance 250mW et de durée égale à 1ms,
  ○ la deuxième fréquence étant choisie égale à 962 kHz,

- Acquérir 405 un deuxième signal réfléchi de contrôle :

  ○ ici encore, l'acquisition du deuxième signal réfléchi peut consister à mesurer une « *puissance* absorbée » par le transducteur, ou un « taux de réflexion », ou une impédance électrique, ou toute autre information représentative d'une puissance consommée par le transducteur,

- Traiter 406 le deuxième signal réfléchi de contrôle pour détecter la présence d'un liquide dans le dispositif ultrasonore :

  ○ une information représentative de la puissance consommée par le transducteur (extraite du deuxième signal réfléchi) est comparée à un deuxième seuil prédéfini correspondant à 64% de la puissance du deuxième signal de contrôle (soit 160mW dans le cas d'un deuxième signal de contrôle de 250mW) :

    ▪ si l'information représentative de la puissance consommée est inférieure au deuxième seuil, alors le milieu s'étendant en regard de la face avant du transducteur est un gaz (i.e. détection d'une bulle de gaz entre le transducteur et le tissu à traiter),
    ▪ sinon, le milieu s'étendant en regard de la face avant du transducteur est un liquide ou du tissu (absence de

**14**

bulle de gaz).

### 4.2.1.3. Détection d'un défaut de fonctionnement d'un transducteur

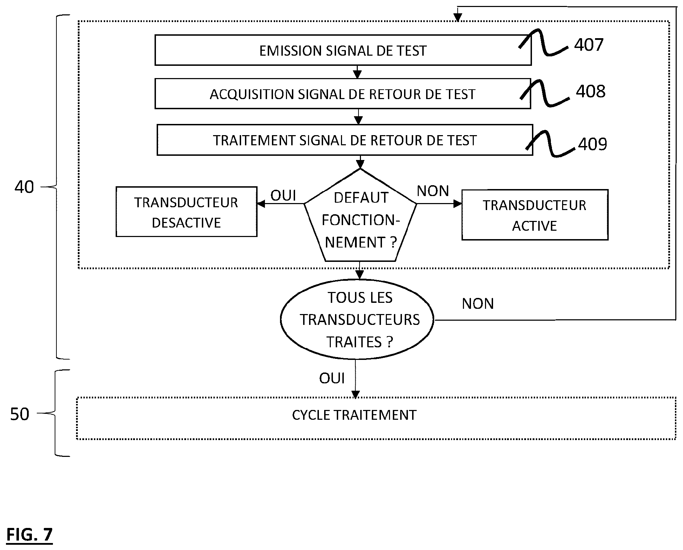

En référence à la figure 7, l'étape de détection d'un défaut de fonctionnement comporte les sous-étapes consistant à :

- Emettre 407 un signal de test à une fréquence de contrôle nulle,
- Acquérir 408 un signal réfléchi de test, cette acquisition du signal réfléchi peut consister à mesurer une *« puissance absorbée »* par le transducteur, ou un « taux de réflexion », ou une impédance électrique, ou toute autre information représentative d'une puissance consommée par le transducteur,
- Traiter 409 le signal réfléchi de test pour détecter un défaut de fonctionnement d'un transducteur : une information représentative de la puissance consommée par le transducteur (extraite du signal réfléchi de test) est comparée à des premier et deuxième seuils de test prédéfinis :

  - si l'information représentative de la puissance consommée est inférieure au premier seuil de test (i.e. impédance nulle ou trop faible), alors le transducteur présente un court-circuit,
  - si l'information représentative de la puissance consommée est supérieure au deuxième seuil de test (i.e. impédance trop forte ou infinie), alors la connexion entre le transducteur et la carte électronique présente un circuit ouvert,
  - sinon, le transducteur fonctionne correctement (absence de défaut de fonctionnement.

**Cette étape** de détection d'un défaut de fonctionnement est réalisée successivement sur chaque transducteur du dispositif ultrasonore. Les transducteurs pour lesquels un défaut de fonctionnement a été détecté sont désactivés tandis que les transducteurs ne présentant aucun défaut de fonctionnement sont activés.

### 4.2.2. Cycle de traitement

Pendant chaque cycle de traitement, le procédé comprend une troisième étape de détection de la présence d'un fluide dans le dispositif ultrasonore.

Cette étape de détection est mise en œuvre avant chaque étape d'émission d'ultrasons de traitement par le dispositif ultrasonore. Ainsi, avant chaque étape consistant à alimenter le (ou les) transducteur(s) en énergie électrique pour induire la génération d'ultrasons de traitement, l'étape de détection de la présence d'un fluide est mise en œuvre.

La troisième étape de détection de la présence d'un fluide comprend les sous-étapes consistant à :

- Emettre 501 un troisième signal de contrôle à une troisième fréquence de contrôle $F_2$ :

  ◦ le troisième signal de contrôle consiste par exemple en un signal impulsionnel de puissance 500mW et de durée égale à 100 $\mu$s,
  ◦ la troisième fréquence étant choisie égale à 600 kHz,

- Mesurer 502 un troisième signal réfléchi de contrôle correspondant à la portion du troisième signal de contrôle n'ayant pas été absorbée par le dispositif ultrasonore,
- Traiter 503 le troisième signal réfléchi de contrôle pour détecter la présence d'un liquide dans le dispositif ultrasonore : une information représentative de la puissance consommée est comparée à un troisième seuil prédéfini correspondant à 40% de la puissance du troisième signal de contrôle (soit 200mW dans le cas d'un troisième signal de contrôle de 500mW) :

  ◦ si l'information représentative de la puissance consommée est inférieure au troisième seuil, alors le dispositif ultrasonore ne contient pas de fluide (i.e. absence de court-circuit),
  ◦ **sinon,** alors le dispositif ultrasonore contient un fluide occasionnant un court-circuit.

En fonction des résultats aux différents test décrit ci-dessus, l'unité de commande 2 commande au dispositif ultrasonore 1 l'émission d'ondes ultrasonores de traitement.

En particulier, si aucun court-circuit n'a été détecté, l'unité de commande 2 alimente le (ou les) transducteur(s) 12 activés du dispositif ultrasonore 1 pour lesquels aucun défaut de couplage n'a été détecté (transducteurs correctement raccordés électriquement, et dont la face avant ne s'étend pas en regard d'une bulle de gaz). Cette étape d'alimentation consiste à appliquer à chaque transducteur activé un signal électrique d'alimentation d'une puissance comprise entre 7 et 8 Watts pendant une durée de 24 ms.

A titre indicatif, un tableau résumant les différentes fréquences utilisées pour la mise en œuvre de la phase d'estimation de la qualité de couplage et de la phase de détection d'un défaut de fonctionnement d'un transducteur est donné ci-dessous.

**Table 1: tableau des fréquences et des tests**

| fréquence | Valeur | Dénomination | utilisation | détails |
|---|---|---|---|---|
| $F_1$ | 1MHz | Fréquence de travail | Emission des ultrasons vers le tissu | choisie dans la bande de fréquence utile du transducteur 12 |
| $F_2$ | 600kHz | troisième fréquence de contrôle ou fréquence de contrôle de courant de fuite | Détection défaut chambre de connexion | Si présence de liquide dans la chambre de connexion (résistance parasite basse, le transducteur ne consomme pas à cette fréquence, la consommation est due à la résistance parasite basse) |
| fréquence | Valeur | Dénomination | utilisation | détails |
| $F_3$ | 850kHz | première fréquence de contrôle | Détection présence transducteur ; raccordement électrique | Le transducteur résonne dans l'air et dans l'eau indépendamment du couplage air ou tissu (avant ou une fois implantée) |
| $F_4$ | 960 à 1MHz | Fréquence de contrôle de gaz | Détection Couplage acoustique | L'impédance vue par l'unité de commande est distincte suivant si le transducteur est couplé à du gaz ou du tissu (uniquement sous TX1) |
| $F_0$ | 0 (continu) | Tension continue | Test du transducteur | a) Court-circuit franc transducteur (impédance nulle, quand le transducteur est activée) b) Détection défaut chambre de connexion (impédance trop faible si présence de liquide), complète test à $F_2$ |

Le lecteur appréciera que les fréquences et seuils utilisés pour l'estimation de la qualité du couplage acoustique et pour la détection d'un défaut de fonctionnement d'un transducteur peuvent :

- être identiques pour tous les dispositifs ultrasonores, ou
- être individualisées pour chaque dispositif ultrasonore.

Cette individualisation permet de tenir compte des éventuelles variations existants entre les performances des différents transducteurs, variation qui peuvent être liées aux tolérances de fabrication des transducteurs (variations de la rugosité de surface ou de l'épaisseur de chaque transducteur, etc.).

*5. Conclusions*

Le procédé décrit précédemment permet d'évaluer la qualité du couplage acoustique entre le dispositif ultrasonore et le tissu à traiter. Il permet également de détecter un éventuel défaut de fonctionnement d'un transducteur.

Il est ainsi possible de limiter les risques d'inefficacité du traitement liée par exemple :

- d'une bulle de gaz entre un (ou plusieurs) transducteur(s) et le tissu à traiter, et/ou
- à un court-circuit dans le dispositif ultrasonore dû à une fuite de liquide au niveau de sa borne de connexion.

La détection de tels défauts permet d'avertir le praticien afin qu'il puisse mettre en œuvre des solutions permettant de corriger ces défauts.

Le lecteur aura compris que de nombreuses modifications peuvent être apportées à l'invention décrite précédemment sans sortir matériellement des nouveaux enseignements et des avantages décrits ici.

Par conséquent, toutes les modifications de ce type sont destinées à être incorporées à l'intérieur de la portée des revendications jointes.

## Revendications

1. Appareil de traitement d'une pathologie comprenant :

 - un dispositif ultrasonore (1) comportant au moins un transducteur (12) apte à générer des ondes ultrasonores, le transducteur ayant une face avant destinée à être positionnée en regard d'un milieu cible,
 - une unité de commande (2) distante pour déterminer et contrôler des paramètres de fonctionnement du dispositif ultrasonore (1), et lui délivrer de l'électricité pendant au moins un cycle de traitement (50), chaque cycle de traitement (50) étant précédé d'un cycle d'attente (40),
 - des moyens de connexion électrique (31, 32) entre le dispositif ultrasonore (1) et l'unité de commande (2),

 l'unité de commande (2) étant programmée pour mettre en œuvre une phase d'estimation de la qualité d'un couplage acoustique entre le dispositif ultrasonore et le milieu cible, ladite phase d'estimation comprenant :

 - l'émission par l'unité de commande, d'au moins un signal de contrôle, chaque signal de contrôle ayant un fréquence respective,
 - la mesure par l'unité de commande, d'au moins un signal réfléchi, chaque signal réfléchi correspondant à un signal de contrôle respectif,
 - le traitement du signal réfléchi pour détecter :

 ◦ soit la présence d'un liquide dans le dispositif ultrasonore,
 ◦ soit la présence d'un matériau réfléchissant, tel qu'une bulle de gaz, entre ledit et au moins un transducteur et le milieu cible

 ***caractérisé en ce que*** la phase d'estimation comprend une étape de détection de la présence de liquide dans le dispositif ultrasonore, ladite étape incluant les sous-étapes suivantes :

 - l'émission par l'unité de commande, d'un signal de contrôle de courant de fuite à une fréquence de contrôle de courant de fuite,
 - la mesure par l'unité de commande, d'un signal réfléchi de contrôle de courant de fuite correspondant à la portion du signal de contrôle de courant de fuite n'ayant pas été absorbée par le dispositif ultrasonore,
 - le traitement du signal réfléchi de contrôle de courant de fuite pour détecter la présence d'un liquide dans le dispositif ultrasonore.

2. Appareil de traitement selon la revendication 1, ***dans lequel*** la fréquence de contrôle de courant de fuite est une fréquence n'appartenant pas à une plage de fréquence de fonctionnement du transducteur, notamment une fréquence de l'ordre de 600 kHz pour un transducteur dont la fréquence de travail est égale à 1 MHz.

3. Appareil de traitement selon la revendication 1, ***dans lequel*** la phase d'estimation comprend une étape de détection de la présence d'une bulle de gaz, ladite étape incluant les sous-étapes suivantes :

- l'émission par l'unité de commande, d'un signal de contrôle de gaz à une fréquence de contrôle de gaz,
- la mesure par l'unité de commande, d'un signal réfléchi de contrôle de gaz correspondant à la portion du signal de contrôle de gaz n'ayant pas été absorbée par le dispositif ultrasonore,
- le traitement du signal réfléchi de contrôle de gaz pour détecter la présence d'une bulle de gaz entre le transducteur et le milieu cible.

4. Appareil de traitement selon la revendication 3, *dans lequel* la fréquence de contrôle d'une bulle de gaz est une fréquence appartenant à une plage de fréquence de fonctionnement du transducteur, plus précisément une fréquence supérieure à 90% d'une fréquence de travail du transducteur, notamment une fréquence de l'ordre de 962kHz pour un transducteur dont la fréquence de travail est égale à 1 MHz.

5. Appareil de traitement selon l'une quelconque des revendications 3 ou 4, *dans lequel* l'étape de détection de la présence d'une bulle de gaz est mise en œuvre pour chaque transducteur pendant au moins un cycle d'attente, ladite étape incluant en outre une étape consistant à :

   - activer chaque transducteur pour lequel aucune bulle de gaz n'a été détectée, les transducteurs activés étant aptes à être alimentés en énergie électrique pour la génération d'ondes ultrasonores de traitement pendant au moins un cycle de traitement postérieur audit et au moins un cycle d'attente,
   - désactiver chaque transducteur pour lequel une bulle de gaz a été détectée, les transducteurs désactivés n'étant pas alimentés en énergie électrique lors du cycle de traitement postérieur audit et au moins un cycle d'attente.

6. Appareil de traitement selon l'une quelconque des revendications précédentes, *dans lequel* chaque séance de traitement comprend une pluralité de cycles de traitement pendant lesquels le dispositif émet des ondes ultrasonores de traitement vers un tissu à traiter, chaque cycle de traitement étant précédé d'un cycle d'attente, l'unité de commande (2) étant programmée pour mettre en œuvre :

   - l'étape de détection de la présence d'une bulle de gaz pendant chaque cycle d'attente,
   - l'étape de détection de la présence d'un liquide pendant chaque cycle de traitement.

7. Appareil de traitement selon les revendications 1 et 3 prises en combinaison, *dans lequel* les étapes de détection de la présence de liquide et de gaz sont mises en œuvre séquentiellement, l'étape de détection de la présence de liquide étant mise en œuvre postérieurement à l'étape de détection de la présence d'une bulle de gaz.

8. Appareil de traitement selon l'une quelconque des revendications précédentes, *dans lequel* le dispositif ultrasonore comporte un boitier dans lequel chaque transducteur est logé, le boitier incluant un fond en regard de la face avant de chaque transducteur, le fond étant constitué en Poly-Ether-Ether-Ketone, l'épaisseur du fond étant, pour une fréquence de travail du transducteur égale à 1MHz, comprise entre 0.3 mm et 0.8 mm, préférentiellement comprise entre 0.3mm et 0.6 mm, et encore plus préférentiellement sensiblement égale à 0,4mm$\pm$0.05 mm.

9. Appareil de traitement selon la revendication 1, *dans lequel* le dispositif ultrasonore comporte une carte électronique sur laquelle chaque transducteur est connecté électriquement, l'unité de commande (2) étant en outre programmée pour mettre en œuvre une phase de détection d'un défaut de fonctionnement de chaque transducteur du dispositif ultrasonore, ladite phase de détection comprenant :

   - l'émission par l'unité de commande, d'au moins un signal de test ayant un fréquence nulle,
   - la mesure par l'unité de commande, d'au moins un signal réfléchi de test,
   - le traitement du signal réfléchi de test pour détecter :

      ◦ soit un court-circuit entre la carte électronique et un transducteur,
      ◦ soit un défaut de connexion électrique entre la carte électronique et un transducteur.

**Patentansprüche**

1. Vorrichtung zur Behandlung einer Pathologie, umfassend:

   - eine Ultraschallvorrichtung (1), die mindestens einen Wandler (12) umfasst, der dazu geeignet ist, Ultraschallwellen zu erzeugen, wobei der Wandler eine Vorderseite aufweist, die dazu bestimmt ist, gegenüberliegend zu einer Zielumgebung positioniert zu sein,
- eine Fernsteuereinheit (2) zum Bestimmen und Kontrollieren von Betriebsparametern der Ultraschallvorrichtung (1) und zu deren Versorgung mit Elektrizität während mindestens eines Behandlungszyklus (50), wobei jedem Behandlungszyklus (50) ein Wartezyklus (40) vorangeht,
- Mittel zur elektrischen Verbindung (31, 32) zwischen der Ultraschallvorrichtung (1) und der Steuereinheit (2), wobei die Steuereinheit (2) dazu programmiert ist, eine Phase zur Schätzung der Qualität einer akustischen Kopplung zwischen der Ultraschallvorrichtung und der Zielumgebung durchzuführen, wobei die Schätzungsphase umfasst:
- das Senden, durch die Steuereinheit, mindestens eines Kontrollsignals, wobei jedes Kontrollsignal eine jeweilige Frequenz aufweist,
- das Messen, durch die Steuereinheit, mindestens eines reflektierten Signals, wobei jedes reflektierte Signal einem jeweiligen Kontrollsignal entspricht,
- das Verarbeiten des reflektierten Signals zum Erkennen:

  ○ entweder des Vorhandenseins einer Flüssigkeit in der Ultraschallvorrichtung,
  ○ oder des Vorhandenseins eines reflektierenden Materials, wie einer Gasblase, zwischen dem mindestens einen Wandler und der Zielumgebung,

**dadurch gekennzeichnet, dass** die Schätzungsphase einen Schritt zum Erkennen des Vorhandenseins von Flüssigkeit in der Ultraschallvorrichtung umfasst, wobei der Schritt die folgenden Teilschritte umfasst:

- das Senden, durch die Steuereinheit, eines Leckstrom-Kontrollsignals mit einer Leckstrom-Kontrollfrequenz,
- das Messen, durch die Steuereinheit, eines reflektierten Leckstrom-Kontrollsignals, das dem Abschnitt des Leckstrom-Kontrollsignals entspricht, der nicht von der Ultraschallvorrichtung absorbiert wurde,
- das Verarbeiten des reflektierten Leckstrom-Kontrollsignals, um das Vorhandensein einer Flüssigkeit in der Ultraschallvorrichtung zu erkennen.

2. Behandlungsvorrichtung nach Anspruch 1, wobei die Leckstrom-Kontrollfrequenz eine Frequenz ist, die nicht zu einem Betriebsfrequenzbereich des Wandlers gehört, insbesondere eine Frequenz in der Größenordnung von 600 kHz für einen Wandler, dessen Arbeitsfrequenz gleich 1 MHz ist.

3. Behandlungsvorrichtung nach Anspruch 1, wobei die Schätzungsphase einen Schritt zum Erkennen des Vorhandenseins einer Gasblase umfasst, wobei der Schritt die folgenden Teilschritte umfasst:

- das Senden, durch die Steuereinheit, eines Gaskontrollsignals mit einer Gaskontrollfrequenz,
- das Messen, durch die Steuereinheit, eines reflektierten Gaskontrollsignals, das dem Abschnitt des Gaskontrollsignals entspricht, der nicht von der Ultraschallvorrichtung absorbiert wurde,
- das Verarbeiten des reflektierten Gaskontrollsignals, um das Vorhandensein einer Gasblase zwischen dem Wandler und der Zielumgebung zu erkennen.

4. Behandlungsvorrichtung nach Anspruch 3, wobei die Frequenz zur Kontrolle einer Gasblase eine Frequenz ist, die zu einem Betriebsfrequenzbereich des Wandlers gehört, genauer gesagt eine Frequenz, die höher als 90 % einer Arbeitsfrequenz des Wandlers ist, insbesondere eine Frequenz in der Größenordnung von 962 kHz für einen Wandler, dessen Arbeitsfrequenz gleich 1 MHz ist.

5. Behandlungsvorrichtung nach einem der Ansprüche 3 oder 4, wobei der Schritt zum Erkennen des Vorhandenseins einer Gasblase für jeden Wandler während mindestens eines Wartezyklus durchgeführt wird, wobei der Schritt ferner einen Schritt umfasst, der besteht im:

- Aktivieren jedes Wandlers, für den keine Gasblase erkannt wurde, wobei die aktivierten Wandler dazu geeignet sind, während mindestens eines Behandlungszyklus nach dem mindestens einen Wartezyklus mit elektrischer Energie zur Erzeugung von Behandlungsultraschallwellen versorgt zu werden,
- Deaktivieren jedes Wandlers, für den eine Gasblase erkannt wurde, wobei die deaktivierten Wandler während des Behandlungszyklus nach dem mindestens einen Wartezyklus nicht mit elektrischer Energie versorgt werden.

6. Behandlungsvorrichtung nach einem der vorhergehenden Ansprüche, wobei jede Behandlungssitzung eine Vielzahl von Behandlungszyklen umfasst, während denen die Vorrichtung Behandlungsultraschallwellen hin zu einem zu behandelnden Gewebe sendet, wobei jedem Behandlungszyklus ein Wartezyklus vorangeht, wobei die Steuereinheit (2) programmiert ist zum Durchführen:

- des Schritts zum Erkennen des Vorhandenseins einer Gasblase während jedes Wartezyklus,
- des Schritts zum Erkennen des Vorhandenseins einer Flüssigkeit während jedes Behandlungszyklus.

7. Behandlungsvorrichtung nach den Ansprüchen 1 und 3 in Kombination, wobei die Schritte zum Erkennens des Vorhandenseins von Flüssigkeit und von Gas aufeinanderfolgend durchgeführt werden, wobei der Schritt zum Erkennen des Vorhandenseins von Flüssigkeit nach dem Schritt zum Erkennen des Vorhandenseins einer Gasblase durchgeführt wird.

8. Behandlungsvorrichtung nach einem der vorhergehenden Ansprüche, wobei die Ultraschallvorrichtung ein Gehäuse umfasst, in dem jeder Wandler untergebracht ist, wobei das Gehäuse einen Boden umfasst, der der Vorderseite jedes Wandlers gegenüberliegt, wobei der Boden aus Polyetheretherketon besteht, die Dicke des Bodens für eine Arbeitsfrequenz des Wandlers von gleich 1 MHz zwischen 0,3 mm und 0,8 mm beträgt, vorzugsweise zwischen 0,3 mm und 0,6 mm beträgt, und noch bevorzugter im Wesentlichen gleich 0,4 mm $\pm$ 0,05 mm ist.

9. Behandlungsvorrichtung nach Anspruch 1, wobei die Ultraschallvorrichtung ferner eine elektronische Karte umfasst, an der jeder Wandler elektrisch verbunden ist, wobei die Steuereinheit (2) ferner dazu programmiert ist, eine Phase zur Erkennung eines Betriebsfehlers jedes Wandlers der Ultraschallvorrichtung durchzuführen, wobei die Erkennungsphase umfasst:

- das Senden, durch die Steuereinheit, mindestens eines Testsignals, das eine Frequenz von Null aufweist,
- das Messen, durch die Steuereinheit, mindestens eines reflektierten Testsignals,
- das Verarbeiten des reflektierten Testsignals, um zu erkennen:

  ◦ entweder einen Kurzschluss zwischen der elektronischen Karte und einem Wandler,
  ◦ oder einen elektrischen Verbindungsfehler zwischen der elektronischen Karte und einem Wandler.

**Claims**

1. An apparatus for treating a pathology comprising:

   - an ultrasonic device (1) including at least one transducer (12) able to generate ultrasonic waves, the transducer having a front face intended to be positioned facing a target medium,
   - a remote control unit (2) for determining and monitoring operating parameters of the ultrasonic device (1), and supplying it with electricity during at least one treatment cycle (50), each treatment cycle (50) being preceded by a wait cycle (40),
   - electrical connection means (31, 32) between the ultrasonic device (1) and the control unit (2),

   the control unit (2) being programmed to implement an estimation phase of the quality of an acoustic coupling between the ultrasonic device and the target medium, said estimation phase comprising:

   - the emission, by the control unit, of at least one monitoring signal, each monitoring signal having a respective frequency,
   - the measurement, by the control unit, of at least one reflected signal, each reflected signal corresponding to a respective monitoring signal,
   - the processing of the reflected signal to detect:

     ◦ either the presence of a liquid in the ultrasonic device,
     ◦ or the presence of a reflective material, such as a gas bubble, between said and at least one transducer and the target medium

   **characterised in that** the estimation phase comprises a step of detecting the presence of liquid in the ultrasonic device, said step including the following sub-steps:

- the emission, by the control unit, of a leakage current monitoring signal at a leakage current monitoring frequency,
- the measurement, by the control unit, of a reflected leakage current monitoring signal corresponding to the portion of the leakage current monitoring signal that has not been absorbed by the ultrasonic device,
- the processing of the reflected leakage current monitoring signal to detect the presence of a liquid in the ultrasonic device.

2. The treatment apparatus according to claim 1, *wherein* the leakage current monitoring frequency is a frequency that does not belong to an operating frequency range of the transducer, in particular a frequency on the order of 600 kHz for a transducer whose working frequency is equal to 1 MHz.

3. The treatment apparatus according to claim 1, *wherein* the estimation phase comprises a step of detecting the presence of a gas bubble, said step including the following sub-steps:

- the emission, by the control unit, of a gas monitoring signal at a gas monitoring frequency,
- the measurement, by the control unit, of a reflected gas monitoring signal corresponding to the portion of the gas monitoring signal that has not been absorbed by the ultrasonic device,
- the processing of the reflected gas monitoring signal to detect the presence of a gas bubble between the transducer and the target medium.

4. The treatment apparatus according to claim 3, *wherein* the gas bubble monitoring frequency is a frequency that belongs to an operating frequency range of the transducer, more specifically a frequency greater than 90% of a working frequency of the transducer, in particular a frequency on the order of 962 kHz for a transducer whose working frequency is equal to 1 MHz.

5. The treatment apparatus according to any one of claims 3 or 4, *wherein* the step of detecting the presence of a gas bubble is implemented for each transducer during at least one wait cycle, said step further including a step consisting in:

- activating each transducer for which no gas bubble has been detected, the activated transducers being able to be supplied with electrical energy for the generation of ultrasonic treatment waves during at least one treatment cycle subsequent to said and at least one wait cycle,
- deactivating each transducer for which a gas bubble has been detected, the deactivated transducers not being supplied with electrical energy during the treatment cycle subsequent to said and at least one wait cycle.

6. The treatment apparatus according to any one of the preceding claims, *wherein* each treatment session comprises a plurality of treatment cycles during which the device emits ultrasonic treatment waves towards a tissue to be treated, each treatment cycle being preceded by a wait cycle, the control unit (2) being programmed to implement:

- the step of detecting the presence of a gas bubble during each wait cycle,
- the step of detecting the presence of a liquid during each treatment cycle.

7. The treatment apparatus according to claims 1 and 3 taken in combination, *wherein* the steps of detecting the presence of liquid and gas are implemented sequentially, the step of detecting the presence of liquid being implemented subsequently to the step of detecting the presence of a gas bubble.

8. The treatment apparatus according to any one of the preceding claims, *wherein* the ultrasonic device includes a casing in which each transducer is housed, the casing including a bottom facing the front face of each transducer, the bottom being made of Poly-Ether-Ether-Ketone, the thickness of the bottom being comprised, for a working frequency of the transducer equal to 1 MHz, between 0.3 mm and 0.8 mm, preferably comprised between 0.3 mm and 0.6 mm, and even more preferably substantially equal to 0.4 mm $\pm$ 0.05 mm.

9. The treatment apparatus according to claim 1, *wherein* the ultrasonic device includes an electronic card on which each transducer is electrically connected, the control unit (2) further being programmed to implement a detection phase of an operating fault of each transducer of the ultrasonic device, said detection phase comprising:

- the emission, by the control unit, of at least one test signal having a zero frequency,
- the measurement, by the control unit, of at least one reflected test signal,

- the processing of the reflected test signal to detect:

  ◦ either a short circuit between the electronic card and a transducer,
  ◦ or an electrical connection fault between the electronic card and a transducer.

**FIG. 1**

**FIG. 2**

**FIG. 3**

EMISSION SIGNAL DE CONTROLE ⎞ 411

ACQUISITION SIGNAL DE RETOUR ⎞ 412

TRAITEMENT SIGNAL DE RETOUR ⎞ 413

NON — QUALITE OK ? — OUI

TRANSDUCTEUR DESACTIVE

TRANSDUCTEUR ACTIVE

**FIG. 4**

Puissance active mesurée par le générateur (250mW émis)

Puissance active (mW)

300
250
200
150
100
50
0
-50

0.5     Fréquence (en MHz)     1.5     2

F2

F3

F4

F1

+ Puissance mesurée dans l'eau (±2*std)
° Puissance mesurée dans l'air (±2*std)

**FIG. 5**

**FIG. 6**

EMISSION SIGNAL DE TEST — 407

ACQUISITION SIGNAL DE RETOUR DE TEST — 408

TRAITEMENT SIGNAL DE RETOUR DE TEST — 409

40

TRANSDUCTEUR DESACTIVE

OUI ← DEFAUT FONCTION-NEMENT ? → NON

TRANSDUCTEUR ACTIVE

TOUS LES TRANSDUCTEURS TRAITES ? — NON

OUI

50

CYCLE TRAITEMENT

**FIG. 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018007500 A
- WO 2020013868 A
- WO 2018185767 A
- EP 0050040 A
- EP 2539021 A